# EUROPEAN PATENT APPLICATION

(11) **EP 4 579 609 A1**
(43) Date of publication of application: **02.07.2025**
(21) Application number: 24219433.0
(22) Date of filing: 12.12.2024
(51) Int. Cl.: G06V 10/62, G06V 10/764, G06V 20/40, G06V 40/20, A61B 5/11, A61B 5/00, G06V 20/52

(54) **FOOTBALL ACTIVITY CLASSIFICATION**

(30) Priority: 29.12.2023 US 202318400989
(71) Applicant: adidas AG, 91074 Herzogenaurach (DE)
(72) Inventor: DUEMLER, Burkhard, Herzogenaurach 91074 (DE)
(74) Representative: Bardehle Pagenberg Partnerschaft mbB Patentanwälte Rechtsanwälte

(57) **Abstract**

A method of determining an event participated in by an athlete includes receiving, at a computing device, a plurality of motion determinations generated by a monitor from motion data captured from the athlete's motions during a monitoring window. The motion determinations include actions performed by the athlete, performance metrics of the athlete, or both. The method also includes classifying, by use of a machine learning model stored on the computing device, which event among a plurality of predetermined events the athlete participated in during the monitoring window based at least in part on the plurality of motion determinations.

## Description

### FIELD

The described embodiments generally relate to application of a machine learning model to determine which event among a plurality of predetermined possible events an athlete participates in based on determinations regarding the athlete's activities derived from motion data captured from the athlete's motion.

### BACKGROUND

It has become common practice in the field of professional athletics to record athletes' activity both within matches and during preparation for matches for the purpose of analyzing the athletes' performance and optimize training. Recorded information for this purpose can include video footage of an area in which athletes are active as well as measurements of particular quantitative factors of individual athletes. For example, athletes may wear motion sensors or biometric sensors while engaged in a match or when training to facilitate analysis of the athlete's individual performance.

The significant amount of data generated in relation to a team of athletes can prove difficult to manage. Records are not readily identified as being acquired during a match, during training, or during other events unless manually sorted into such categories. Data from different sources, such as video footage and motion sensor data, are not synchronized upon creation, and therefore cannot be considered together unless collated by a human analyst. Thus, attempting to derive more than cursory insights from the types and quantities of data now available regarding athletes' performance can be a significantly time consuming endeavor.

### BRIEF SUMMARY

Systems and methods herein apply machine learning technology to identify which type of activity an athlete was participating in at a time when records of the athlete's performance were generated. According to some embodiments, a system can comprise a wearable monitor configured to measure an athlete's motion, thereby capturing motion data from the athlete's motion, and generate motion determinations based on the motion data. In some embodiments, the wearable monitor is configured with a machine learning model for generating the motion determinations based on the motion data. The motion classifications can comprise individual actions the athlete performs, metrics of the athlete's performance, or both. According to some further embodiments, a machine learning model can analyze a set of the motion classifications and classify the motion data, the motion determinations, or both, according to which among a plurality of predetermined events the athlete was participating in when the motion data were captured. According to some further embodiments, the machine learning model may run on a computing device that is separate from the wearable monitor.

In some embodiments, use of a machine learning model to classify which event or events the athlete participated in, and determine when the athlete's participation in individual events began and ended, based on information reported by a wearable monitor can expedite analysis of the athlete's performance, including providing improved activity recognition and classification. For example, after the machine learning model divides data into warmup and match data, a system can provide analytical tools to facilitate analysis of the warmup data and math data across multiple days to assess what warmup types result in better in-match performance. The use of the machine learning model can also expedite synchronizing information from a wearable monitor with video footage by the machine learning model's identification of start or end times for specific events within the information and footage without need for human oversight.

Aspects of the present disclosure are directed to a method of determining an event participated in by an athlete. The method can comprise receiving, at a computing device, a plurality of motion determinations generated based on motion data captured from motion data of the athlete during a monitoring window. The motion determinations can comprise at least one of an action performed by the athlete and performance metrics of the athlete. The method can comprise classifying, by use of a machine learning model stored on the computing device and based on the motion determinations, an event based at least in part on the plurality of motion determinations. The event can represent a classification of the plurality of motion determinations. The method can comprise generating a graphical user interface visualizing the event in relation to a time-related parameter.

In some embodiments according to the foregoing, the method can comprise generating, by the computing device, a timeline of events participated in by the athlete based at least in part on a set of motion determinations comprising the plurality of motion determinations by applying the machine learning model to the set of motion determinations.

In some embodiments according to any of the foregoing, generating the timeline of events can comprise classifying individual motion determinations among the plurality of motion determinations as being generated from motion data captured during individual events among the timeline of events.

In some embodiments according to any of the foregoing, the timeline of events participated in by the athlete can be an output timeline. The method can comprise, before the receiving step, training the machine learning model to identify events athletes participate in by submitting training timelines to the machine learning model. Each training timeline can comprise a plurality of sample motion determinations and indications of when sample events occurred.

In some embodiments according to any of the foregoing, the indications of when sample events occurred can include event type tags associated with sample motion determinations among the plurality of sample motion determinations.

In some embodiments according to any of the foregoing, the timeline can be a filtered timeline. Generating the timeline can comprise generating an unfiltered timeline of events participated in by the athlete based in part on the set of motion determinations by applying the machine learning model to the set of motion determinations. Generating the timeline can also comprise filtering the unfiltered timeline of events by changing start times of individual events within the timeline of events to comply with filtering rules.

In some embodiments according to any of the foregoing, the filtering rules can comprise possible durations for events among a plurality of predetermined events.

In some embodiments according to any of the foregoing, the plurality of predetermined events can comprise exercise, training for a sport, and a match of the sport.

In some embodiments according to any of the foregoing, the method can comprise training the machine learning model to classify events participated in based on motion determinations. The training can comprise creating a plurality of test motion determinations based on motions of a test athlete during a test window. The training can also comprise using the machine learning model to output a test event classification of which event among the plurality of predetermined events the test athlete participated in during the test window based on the plurality of test motion determinations. The training can also comprise correcting the test event classification based on a record of what event the test athlete participated in during the test window.

In some embodiments according to any of the foregoing, the method can comprise determining, by the computing device, a role of the athlete in a team sport based at least in part on the plurality of motion determinations by applying the machine learning model to the plurality of motion determinations.

In some embodiments according to any of the foregoing, the motion determinations can comprise actions performed by the athlete. The actions performed by the athlete can comprise any one or any combination of a kick, a step, dribbling a ball, and running.

In some embodiments according to any of the foregoing, the motion determinations can comprise performance metrics. The performance metrics can comprise any one or any combination of distance traveled, travel speed, and kick force.

In some embodiments according to any of the foregoing, the classifying, by use of the machine learning model, which event among a plurality of predetermined events the athlete participated in during the monitoring window can be further based on video footage of the athlete during the monitoring window.

In some embodiments according to any of the foregoing, the method can comprise training the machine learning model to classify events participated in by athletes based on video footage. The training can comprise creating tagged footage by tagging training video footage of athletes participating in events among the plurality of predetermined events with start times of the events among the plurality of predetermined events. The training can also comprise training the machine learning model on the tagged footage to recognize participation in the events among the plurality of predetermined events.

Some aspects of the present disclosure are directed to a system. The system can comprise a wearable sensor configured to measure motion of a wearer of the wearable sensor. The system can also comprise a controller configured to generate motion determinations from motion data captured by the wearable sensor. The motion determinations can comprise either or both of actions performed by the wearer and performance metrics of the wearer. The system can also comprise a computing device comprising a processor and a non-transitory computer readable medium, wherein the non-transitory computer readable medium carries instructions that, when read by the processor, cause the processor to classify which event among a plurality of predetermined events the wearer participated in while the motion data were captured by the wearable sensor based at least in part on a plurality of the motion determinations.

In some embodiments according to any of the foregoing, the wearable sensor can be configured to be integrated into an article of wear.

In some embodiments according to any of the foregoing, the article of wear can comprise a shoe.

In some embodiments according to any of the foregoing, the system can comprise a wearable monitor that comprises the wearable sensor and the controller.

In some embodiments according to any of the foregoing, the computing device can be remote from the wearable sensor.

In some embodiments according to any of the foregoing, the computing device can comprise any one or any combination of a smart device, a laptop computer, a desktop computer, or a cloud computing system.

In some embodiments according to any of the foregoing, the instructions can be part of a machine learning model.

In some embodiments according to any of the foregoing, the system can comprise an object sensor configured to be integrated into a sports object and to measure motion of the sports object, wherein the instructions, when read by the processor, cause the processor to classify which event among the plurality of predetermined events the wearer participated in while the motion data were captured by the wearable sensor based in part on measurements made by the object sensor.

In some embodiments according to any of the foregoing, events among the plurality of predetermined events can comprise any one or any combination of warmup, training, and a match.

In some embodiments according to any of the foregoing, the instructions, when read by the processor, can cause the processor to classify which event among the plurality of predetermined events the wearer participated in based in part on rules of a sport.

In some embodiments according to any of the foregoing, the rules of the sport can comprise match duration.

In some embodiments according to any of the foregoing, the instructions, when read by the processor, can cause the processor to classify which event among the plurality of predetermined events the wearer participated in based in part on video footage of the wearer.

### BRIEF DESCRIPTION OF THE DRAWINGS/FIGURES

FIG. 1A illustrates a system according to some aspects of the present disclosure.
FIG. 1B illustrates a data flow within the system according to some aspects of the present disclosure
FIG. 2A is a graph of action classifications according to some aspects of the present disclosure.
FIG. 2B is a graph of performance metrics according to some aspects of the present disclosure.
FIG. 2C is a graph of object metrics according to some aspects of the present disclosure.
FIG. 3A is a schematic representation of inputs and outputs for a machine learning model according to some aspects of the present disclosure.
FIG. 3B illustrates an alternative output of a machine learning model according to some aspects of the present disclosure.
FIG. 4 is a flow chart of a training method for a machine learning model according to some aspects of the present disclosure.
FIG. 5 is a flow chart of another training method for a machine learning model according to some aspects of the present disclosure.

### DETAILED DESCRIPTION

The concepts of the present disclosure will be described in detail with reference to embodiments thereof as illustrated in the accompanying drawings. References to "some embodiments", "one embodiment", "an embodiment", "an exemplary embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

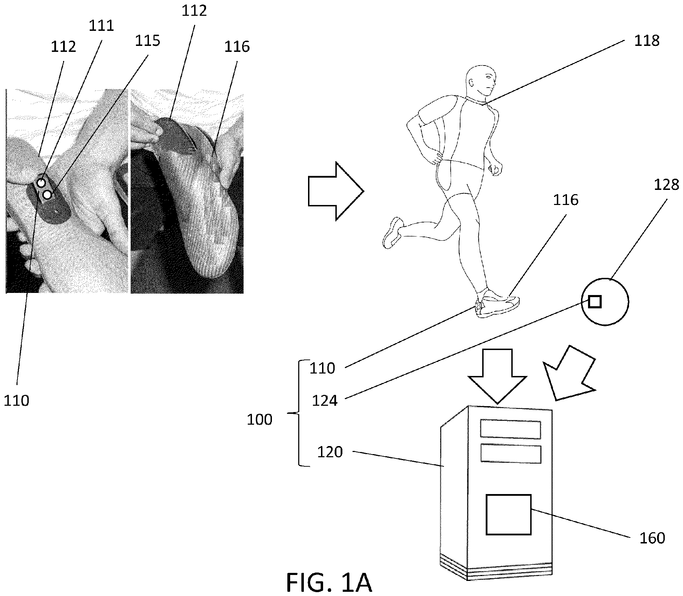

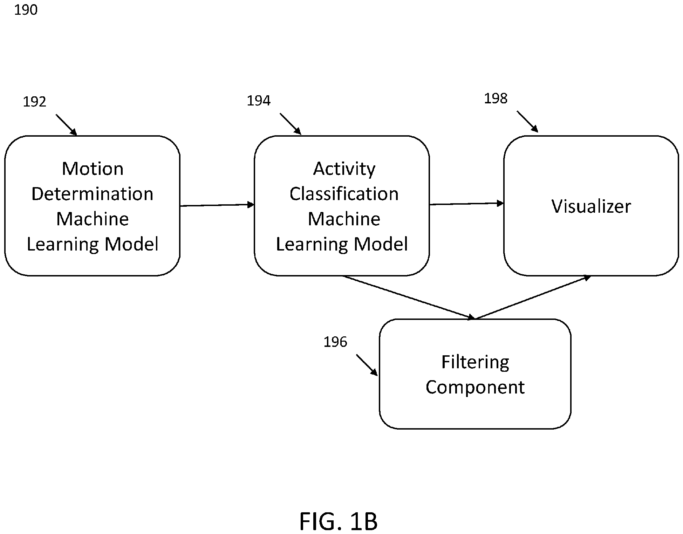

FIG. 1A illustrates a system 100 comprising an athlete monitor 110 and a computing device 120. Athlete monitor 110 can be a wearable monitor. Athlete monitor 110 can be configured to generate motion determinations of a wearer, such as athlete 118, of athlete monitor 110 based on motion of the wearer. FIG. 1 shows one athlete 118 and athlete monitor 110 for simplicity, but system 100 can comprise any number of athlete monitors 110 to be worn by different athletes 118. For example, each member of a team of athletes 118 can wear a respective athlete monitor 110 and all of the athlete monitors 110 may be part of system 100.

Computing device 120 may receive and store motion data from any number of athlete monitors 110 and provides received motion data to a machine learning model 160 trained to classify one or more activities or events in the motion data. For example, machine learning model 160 may be trained to identify, in the motion data, different events such as games, training, or exercising as well as identify different athletes within the motion data. Machine learning model 160 may then provide these classifications to a graphical user interface and/or analysis tools for further processing. As one non-limiting example, motion data may comprise video data (e.g., video of a game, training and/or exercise session) and machine learning model 160 may automatically add visual annotations to the video data to indicate the identified classifications. Exercise, in this particular context, can refer to activities having the primary purpose of improving aspects of an athlete's 118 physical fitness. In contrast, training, in this particular contrast, can refer to activities having the primary purpose of developing an athlete's 118 skills. As another example, machine learning model 160 may be configured to generate a visual report comprising the classifications along with timestamped images or data indicating where the classifications take place within the video data.

Machine learning model 160 according to various embodiments can be any type of machine learning model 160. Thus, machine learning model 160 in some embodiments can be, for example, a neural network.

Machine learning model 160 classifies motion determinations based on motion data provided from monitor 110. These classifications may include one or more events from among a plurality of predetermined types of events. Depending on the desired output, machine learning model 160 may be trained identify different types of events based on the target activity, such as soccer, football, tennis, to name a few examples. For example, for soccer, the different types of events may include activities that do not involve a soccer ball (e.g., jogging, sprinting), training with a soccer ball (e.g, passing drills, penalty kicks, free kicks) and activities that simulate a soccer game. For this disclosure, the terms "event" and "activity" are used interchangeably to refer to classifications that can be applied to motion data.

In some embodiments, machine learning model 160 may be trained using annotated video data. In some embodiments, the video data may be annotated with information such as what portions of the video depict specific activities among a group of predetermined activities or predetermined events, such as, for example, training, a match, or exercise. In further embodiments, the video data may further be annotated with subtypes of any of the predetermined activities or predetermined events, such as, for example, a type of exercise being performed.

In some embodiments, machine learning model 160 may be trained in further view of monitor 110 information streams, such as one or more collections of motion determinations generated by monitors 110. By learning from annotated video data in combination with motion determinations generated by monitors 110 from motion data captured during the events depicted in the annotated video, machine learning model 160 may learn what patterns what patterns of motion determinations tend to appear from motion data captured during each type of event among the plurality of predetermined events. For example, machine learning model 160 according to some embodiments may learn that extended sequences of jogging or sprinting tend to occur during exercise, whereas extended repetition of actions such as passing, penalty kicks, free kicks, and so on tends to occur during training. For example, machine learning model 160 according to some embodiments may learn that uneven mixes of various actions, such as running, kicking, and passing, tend to occur during matches.

In some embodiments, machine learning model 160 is trained to output of events an athlete 118 participated in at the time of capture of motion data submitted to machine learning model 160. For example, machine learning model 160 according to some embodiments is trained to receive a plurality of motion determinations and classify which event or activity an athlete 118 participated in while wearing monitor 110 that generated the motion determinations.

Motion data is captured via athlete monitor 110, which can be a wearable athlete monitor. Athlete monitor 110 can comprise a sensor 111 and a controller 115. Because athlete monitor 110 can be a wearable athlete monitor, sensor 111 can be a wearable sensor. Similarly, controller 115 can be a wearable controller.

Sensor 111 can comprise a motion sensor configured to capture motion data associated with motions of an athlete 118 over a configurable time period. The motion sensor can comprise, for example, any one or any combination of an accelerometer, a magnetometer, a gyroscope, a global positioning system ("GPS"), a pedometer, a posture sensor, an impact sensor, a pressure sensor, and a plurality of any of the foregoing. In further embodiments, sensor 111 can comprise any one or any combination of a heart rate monitor, a temperature sensor, a respiration sensor, a posture sensor, a lactate sensor, and a plurality of any of the foregoing.

Controller 115 can be configured to generate motion determinations from motion data captured by sensor 111. In this disclosure, motion determinations generated by athlete monitor 110 differ from event classifications generated by machine learning model 160. Motion determinations are generated based on motion data provided by sensor 111, while event classifications are generated based on the motion determinations provided by athlete monitor 110.

Because athlete monitor 110 comprises controller 115, athlete monitor 110 according to some embodiments can be configured to generate motion determinations from motion data captured by sensor 111. Motion determinations may comprise either or both of actions performed and performance metrics. In some embodiments, athlete monitor 110 may be configured with a machine learning model to process the motion data captured by sensor 111 and generate the motion determinations. Similarly to machine learning model 160 of computing device, the machine learning model of athlete monitor 110 could be any of a variety of types of machine learning model. In some embodiments, the machine learning model of athlete monitor 110 is a neural network. In some embodiments, motion data captured by athlete monitor 110 can further include timestamps which identifies a time at which the captured motion was recorded. Thus, for example, in some embodiments wherein athlete monitor 110 generates a motion determination that athlete 118 kicked a ball, athlete monitor 110 can further be configured to determine when athlete 118 kicked the ball based on the corresponding timestamps. In another example, in some embodiments wherein athlete monitor 110 generates a motion determination that athlete 118 traveled, athlete monitor 110 can further be configured to determine a distance that athlete 118 traveled during a certain interval of time.

In some embodiments, athlete monitor 110 can be configured to be integrated into an article of wear. The article of wear can comprise, for example, a shoe 116, a wristband, glove, a shirt, a headband, a hat, or any other article of wear. Athlete monitor 110 of the illustrated embodiment can be integrated into shoe 116 by inserting athlete monitor 110 into an insole 112, then disposing insole 112 within shoe 116. Athlete 118 can therefore wear athlete monitor 110 of the illustrated embodiment by wearing shoe 116 while athlete monitor 110 remains inserted in insole 112 and insole 112 remains disposed within shoe 116. Athlete monitor 110 according to other embodiments can be integrated into shoe 116 or other articles of wear by any processes and tools appropriate for the type of article and the type of activity for which athlete monitor 110 is intended.

Computing device 120 can comprise a processor and memory. The memory may store instructions that, when read by the processor, cause the processor to determine, based at least in part on a plurality of the motion determinations, which event among a plurality of predetermined events athlete 118 participated in while performing motions upon which the plurality of motion determinations were based. The instructions stored on the medium can comprise part of the machine learning model 160.

In some embodiments, computing device 120 can be remote from athlete monitor 110. That is, computing device 120 can be a separate physical device from athlete monitor 110. Accordingly, in some embodiments, computing device 120 can comprise, for example, comprises any one or any combination of a smart device, a laptop computer, a desktop computer, and a cloud computing system. In such embodiments, athlete monitor 110 is configured transmit output from its machine learning model (i.e., motion determinations) as input to machine learning model 160 in computing device 120. Thus, within system 100 according to some embodiments, athlete monitor 110 can host a first machine learning model while computing device 120 hosts a second machine learning model 160. In such embodiments, the first machine learning model and second machine learning model 160 fulfill distinct roles at different steps in a process extending from capture of motion data from athlete 118 to ultimately classifying what activities or events the athlete participated in while wearing athlete monitor 110.

In some embodiments, athlete monitor 110 can generate motion determinations and report them to computing device 120. In such embodiments, the machine learning model 160 stored on computing device 120 can then operate on hardware separate from athlete monitor 110 to determine, based in part or entirely on the motion determinations, what event athlete 118 participated in while performing the motions from which the motion determinations were generated.

Athlete monitor 110 according to various embodiments can be configured to communicate with computing device 120 to report motion determinations through any electronic communication processes, hardware, and protocols. In some embodiments, athlete monitor 110 can be configured to report motion determinations to computing device 120 wirelessly. In further embodiments, athlete monitor 110 can be configured to report motion determinations to computing device 120 through a physical electronic connector. In further embodiments, athlete monitor 110 can be configured to report motion determinations to computing device 120 through one or more wires electronically connecting athlete monitor 110 to device 120. Athlete monitor 110 in some embodiments can be configured to report motion determinations to computing device 120 as the motion determinations are made in real time, such as while athlete monitor 110 is being worn by an active athlete 118. Athlete monitor 110 in further embodiments can be configured to report motion determinations to computing device 120 in a batch, such as after athlete has completed participation in one or more events while wearing athlete monitor 110. In further embodiments, athlete monitor 110 can be configured to transmit information to an intermediary, and the intermediary can be configured to transmit information to computing device 120. The intermediary can be, for example, a smart phone, a cloud computing system, or any other computing device. In some embodiments, athlete monitor 110 can transmit the motion determinations to the intermediary, and the intermediary can then transmit the motion determinations to computing device. In further embodiments, athlete monitor 110 can be configured to transmit the motion data to the intermediary, the intermediary can host a machine learning model configured to derive the motion determinations from the motion data, and the intermediary can be configured to transmit the motion determinations derived by the machine learning model to computing device 120. Thus, all processes described herein as being carried out by processor 115 could, in other embodiments, be carried out on an intermediary device instead.

In some embodiments, system 100 can optionally further comprise object monitor 124. Object monitor 124 can be configured to be integrated into a sports object 128. Sports object 128 of the illustrated embodiment is a ball 128. Ball 128 according to various further embodiments can be a ball suitable for any type of sport, including, for example, soccer. Sports object 128 according to further embodiments can comprise, for example, any type of sport ball, any type of sport "stick" (e.g., a baseball bat, hockey stick, golf club, table tennis paddle, or tennis racquet), a sport glove (e.g., a boxing glove), a bicycle, an oar, a ski, a skateboard, or a surfboard, used by an individual (e.g., athlete 118) during an athletic activity.

Similar to athlete monitor 110, object monitor 124 can comprise a controller and a sensor. The sensor can comprise a motion sensor, such as the motion sensors described above with regard to sensor 111 of athlete monitor 110. The controller of object monitor can similarly be configured to generate object determinations based on motion data captured by the sensor of object monitor 124. The object determinations can comprise, for example, movements of object 128, location of object 128, impacts upon object 128, and actions performed upon or with object 128. Object monitor 124 can further be configured to report object determinations to computing device 120 in any of the manners that athlete monitor 110 may communicate with computing device. However, in some embodiments, system 100 can lack object monitor 124.

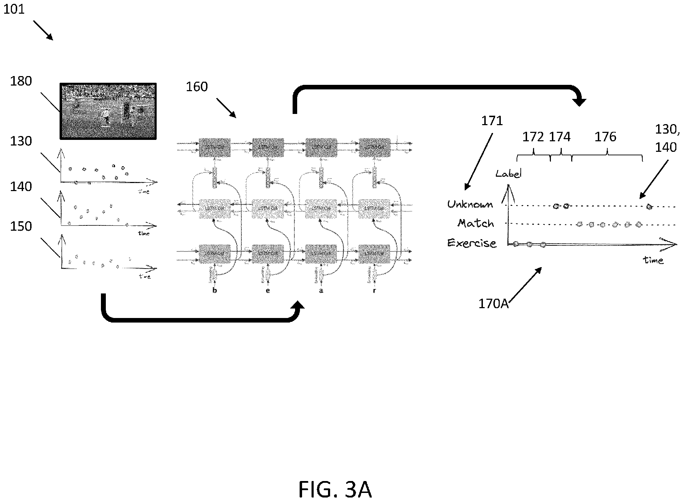

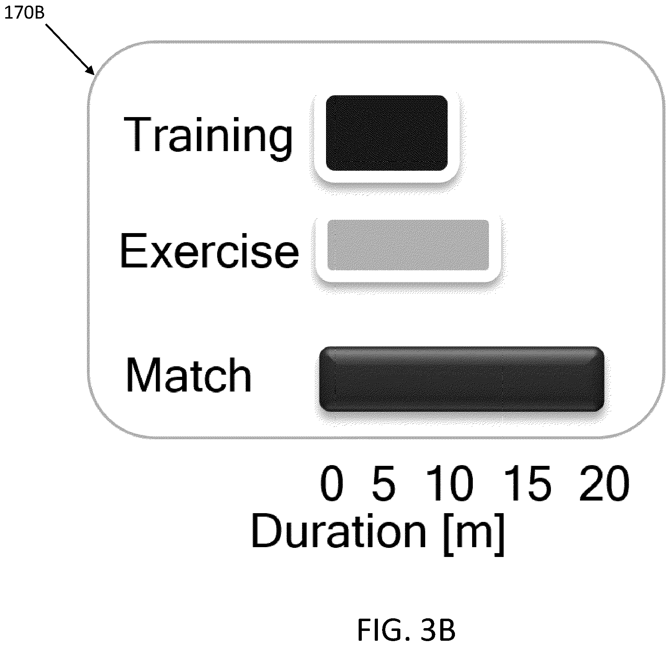

FIG. 1B illustrates an exemplary flow 190 of data within system 100, components of which may be implemented as a motion determination machine learning model 192, an event classification machine learning model 194, filtering component 196, and a visualizer 198. In some embodiments, motion determination machine learning model 192 may be implemented in athlete monitor 110 and event classification machine learning model 194 may be implemented in computing device 120 (e.g., machine learning model 160). Visualizer 198 may be configured to generate visualizations of output from event classification machine learning model 194. Examples of visualizations include arranging the event classifications provided by motion classification machine learning model in a time-based manner, such as in a timeline (e.g., showing a sequence of event classifications over a duration of time, see FIG. 3A) or as a table (e.g., showing the event classifications in relation to each other with respect to how much time the classifications appear within the source data, see FIG. 3B).

In some embodiments, motion determination machine learning model 192 is configured to receive motion data, such as from sensor 111, and generate motion determinations based on the motion data. In some embodiments, there may be separate motion determination machine learning models for different target activities, such as different sports. That is, motion determination machine learning model 192 may be trained to generate motion determinations for a specific sport (e.g., soccer) while there are other motion determination machine learning models for other sports.

In some embodiments, motion determination machine learning models 192 may be implemented into athlete monitor 110 based on user selection. For example, a user may upload a motion determination machine learning model 192 into athlete monitor 110 one day while training for soccer and a different motion determination machine learning model another day while training for football. Accordingly, the types of motion determinations that athlete monitor 110 generates may be customized based on the type of motion determination machine learning model that is installed.

In some embodiments, output of event classification machine learning model 194 is provided to a filtering component 196 for additional processing of the motion classifications based on filtering rules for further organizing the motion classifications.

In some embodiments, visualizer 198 is implemented in a computing device separate from computing device 120. For example, visualizer 198 may be implemented in user device such as a smartphone, laptop, tablet, or personal computer and may be configured to generate and display requested visualizations of the event classifications.

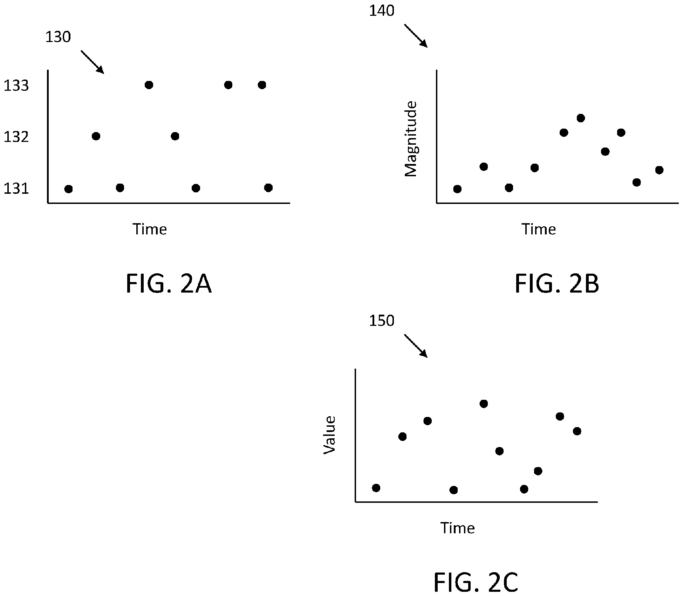

As shown in FIG. 2A, in some embodiments, motion determinations comprise actions 130 performed by athlete 118. Actions 130 performed by athlete 118 and identifiable by controller 115 as motion determinations can comprise instances of actions among a plurality of predetermined action types. Thus, as shown in the illustrated example, controller 115 can be configured to determine when athlete 118 performs an action of a first predetermined action type 131, a second predetermined action type 132, or a third predetermined action type 133. The number of predetermined action types can vary in other embodiments based on the type of activity and annotated video data on which machine learning model 160 was trained. For example, in some embodiments, athlete monitor 110 can be configured to determine only one predetermined action type. In further examples, athlete monitor 110 can be configured to determine two predetermined action types, or any other plural number of predetermined action types. According to some embodiments, the predetermined action types can comprise, for example, a kick, a step, dribbling a ball, running, jogging, and walking. As will be discussed below, a machine learning model on athlete monitor 110 can be trained to detect any number of predetermined action types based on the target activity (e.g., soccer, football, baseball). That is, action types may be predetermined based on the target activity on which a machine learning model is trained. In some embodiments, action type motion determinations can be made further based on an additional layer of processing using information other than information acquired by sensor 111. For example, travel speeds or distances indicative of walking, jogging, or running can differ depending on the age, gender, or size of athlete 118. Thus, in some embodiments, action type motion determinations can be made based on information acquired by sensor 111 in combination with demographic information of the wearer of monitor 110. As one non-limiting example, a travel speed of 10 km/h may be classified as a running action or a jogging action depending on the age of athlete 118. There may be configurable threshold values associated with the additional layer of processing such as a threshold age for certain action type motion determinations. If athlete 118 is 10 years old, 10 km/h may be more defined as running whereas if athlete 118 is 18 years old, 10 km/h may be defined as jogging. The additional layer of processing may include one or more rules and thresholds for defining action type motions, including walking, jogging, running, high-speed running, sprinting. The additional layer of processing may also be configured to perform action type motion determinations that are sport-specific. Such as for soccer, the layer of processing may determine types of kicks that can be based on the speed of the ball. Other parameters, besides age and sport-specific activities that may factor into the additional layer of processing include gender-specific actions.

As a non-limiting example of determining action types, when athlete 118 kicks a ball while wearing monitor 110, sensor 111 may capture motion data from the motion of athlete 118 and report the motion data to controller 115. Controller 115 may process the motion data, determine the measurement to be consistent with kicking a ball, and thereby generate a motion determination that athlete 118 performed a kick. Athlete monitor 110 can also identify a time of occurrence for each action 130 monitor 110 determines from motion data captured by sensor 111.

As shown in FIG. 2B, in some embodiments, motion determinations comprise performance metrics 140 of athlete 118. In further embodiments, motion determinations comprise both actions 130 performed by athlete 118 and performance metrics 140 of athlete 118. In some embodiments, performance metrics 140 comprise quantitative assessments of actions performed by athlete 118. Thus, monitor 110 can determine a magnitude of performance metrics 140. According to some embodiments, performance metrics 140 can comprise any one or any one or any combination of distance traveled, travel speed, and kick force. Thus, for example, when athlete 118 runs while wearing monitor 110, sensor 111 may capture motion data from motion of athlete 118 and report the motion data to controller 115. Controller 115 may process the motion data, determine the motion data to be consistent with athlete 118 travelling at particular speed, and thereby generate a motion determination that athlete 118 is travelling at the particular speed. In some embodiments, controller 115 can further generate motion determinations of what distance athlete 118 has traveled within an amount of time. Athlete monitor 110 can also assign a time to each performance metric 140 indicating when the performance metric 140 was accurate as to athlete's 118 performance. In some embodiments, performance metrics can be segmented into predetermined time intervals so that each motion determination pertaining to certain performance metrics can comprise the value for a performance metric within a discrete time interval. For example, a travel speed performance metric can be segmented into predetermined time intervals such that each motion determination pertaining to travel speed comprises an average travel speed within one of the predetermined intervals. In further examples, a travel distance performance metric can be segmented into predetermined time intervals such that each motion determination pertaining to travel distance comprises a total distance traveled within one of the predetermined time intervals. Time intervals can be, for example, 0.5 seconds, 1.0 seconds, 2.0 seconds, 3.0 seconds, 4.0 seconds, 5.0 seconds, or any other amount of time. In some examples, performance metrics can be used to make motion determinations pertaining to action types. For example, in some embodiments controller 115 can make an action type motion determination of whether athlete 118 was standing, walking, jogging, or running based on performance metrics of travel speed or travel distance for a time interval.

In embodiments wherein system 100 comprises object monitor 124, object monitor 124 can generate object determinations 150. As noted above, object determinations 150 can comprise, for example, movements of object 128, location of object 128, impacts upon object 128, and actions performed upon or with object 128. Thus, object monitor 124 can assign a value to each object determination 150. The nature of the value can depend on the type of object determination 150. For example, in some embodiments, the value can be a type of event, among a plurality of predetermined events, that happened to sports object 128. In further embodiments, the value can be a magnitude of a measured quantity related to sports object 128, such as a magnitude of force applied to sports object 128, a speed of travel of sports object 128, or a rate of acceleration of sports object 128. Object monitor 124 can also assign a time to each object determination 150 indicating a time at which the object determination 150 was accurate as to the state of sports object 128.

As shown in FIG. 3A, motion determinations, such as actions 130, performance metrics 140, or both actions 130 and performance metrics 140, can be submitted to machine learning model 160. Machine learning model 160 can then classify what event among a plurality of predetermined events 171 athlete 118 was engaged in while sensor 111 captured the motion data from which motion determinations (i.e., either or both of actions 130 and performance metrics 140) were generated. The plurality of predetermined events 171 of the illustrated embodiment comprises exercise and a match, such as a match or a game of a sport. The plurality of predetermined events 171 can also comprise an unknown category with which machine learning model 160 can label motion determinations that machine learning model 160 cannot assign to another event type. In further embodiments, the plurality of predetermined events 171 can further comprise warmup for another type of event 171. In further embodiments, the plurality of predetermined events can further comprise training, such as training for soccer or another particular type of sport. In further embodiments, machine learning model 160 can be trained to label motion determinations as being generated from activities occurring in any non-zero number of predetermined events 171. In further embodiments, the plurality of predetermined events 171 can comprise any group of the example predetermined events 171 provided herein. In further embodiments, machine learning model 160 can further be trained to determine a role of athlete 118 in a team sport based on the plurality of motion determinations. For example, in some embodiments, machine learning model 160 can be trained to determine whether athlete 118 was participating in an soccer match as a goal-keeper or an outfield player while wearing monitor 110.

Machine learning model 160 can be trained to classify which event athlete 118 participated based in part on a plurality of consecutive motion determinations. Thus, machine learning model 160 can be trained to classify which event athlete 118 participated in based at least in part on a sequence of events represented by a plurality of consecutive motion determinations. The time during which monitor 110 captures motion data from motion of athlete 118 can be considered a series of monitoring windows, such as monitoring windows 172, 174, and 176, within which respective sequences of events occur that machine learning model 160 would interpret as indicating participation by athlete 118 in a specific type of predetermined event 171. Monitoring windows 172, 174, 176 are presented in FIG. 3A for purposes of illustration, but a period of time during which monitor 110 was used to capture motion data from motion of athlete 118 can be divided into any number of monitoring windows having any proportions to one another. Thus, in some embodiments, a method enabled by system 100 can comprise receiving, at computing device 120, a plurality of motion determinations generated by monitor 110 from motion data captured from motions of athlete 118 during a monitoring window, such as any of monitoring windows 172, 174, 176. The method can further comprise determining, by used of machine learning model 160 stored on computing device 120, which event 171 among the plurality of predetermined events 171 athlete 118 participated in during the monitoring window during which the motion data were captured.

Monitor 110 can acquire enough motion data during each monitoring window 172, 174, 176 for a plurality of motion determinations. Thus, a set of motion determinations presented to machine learning model 160 can comprise multiple pluralities of motion determinations, and each of the pluralities of motion determinations may have been generated by athlete monitor 110 determinations from motion data captured during a different monitoring window. Accordingly, in some embodiments, a method enabled by system 100 can also comprise generating, by the computing device, a timeline 170 of multiple events 171 participated in by athlete 118 based at least in part on a set of motion determinations by applying machine learning model 160 to the set of motion determinations. In the method, the set of motion determinations can comprise one or more pluralities of motion determinations, and each of the one or more pluralities of motion determinations may have been generated from motion data captured during a different single monitoring window. The generated timeline 170 of events 171 can therefore span multiple monitoring windows 172, 174, 176, with machine learning model 160 concluding different events occurred in some monitoring windows than in other monitoring windows. In some embodiments, generating timeline 170 of events 171 comprises classifying individual motion determinations among the plurality of motion determinations as being generated from motion data captured during individual events 171 among the timeline 170 of events 171.

In embodiments wherein system comprises object monitor 124, object determinations 150 can also be submitted to machine learning model 160. Accordingly, in some embodiments, machine learning model 160 can be trained to classify what events 171 athlete 118 participated in based in further part on object determinations 150. However, in some embodiments, machine learning model 160 can be trained to classify what events 171 athlete 118 participated in without object determinations 150.

In some embodiments, video footage 180 of athlete 118 during one or more of the monitoring windows 172, 174, 176 can also be submitted to machine learning model 160. Accordingly, in some embodiments, machine learning model 160 can be trained to classify what events 171 athlete participated in during one or more monitoring windows 172, 174,176 based in further part on video footage of athlete 118 during the one or more monitoring windows 172, 174, 176.

In some embodiments, a timeline 170 can be synchronized to video footage 180 such that each motion determination in timeline 170 corresponds to a portion of video footage 180. Timeline 170 according to some embodiments can therefore be used within a user interface, such as a digital graphical user interface. In some such interfaces, a user may navigate to a point within the video footage 180 by selecting a point along timeline 170 or selecting a motion determination within timeline 170. That is, a graphical user interface according to some embodiments can display multiple motion determinations within a timeline and be configured such that selecting one of the motion determinations causes the device that displays the graphical user interface to further display a portion of video footage 180 corresponding in time to the motion data upon which the selected motion determination is based. In further embodiments, the graphical user interface can indicate the beginning and ends of monitoring windows 172, 174, 176 along the timeline. In some such embodiments, the graphical user interface can be configured such that selecting one of the monitoring windows 172, 174, 176 causes the device that displays the graphical user interface to further display a portion of the video footage 180 corresponding in time to the selected one of the monitoring windows 172, 174, 176. Timeline 170 can be synchronized to video footage 180 even if machine learning model 160 does not generate timeline 170 based in any part on video footage 180. In some embodiments, machine learning model 160 can synchronize timeline 170 with video footage 180.

In some embodiments, outputs from machine learning model 160 can be refined based on external considerations. For example, in some embodiments, outputs from machine learning model 160 can be refined based on classification rules such as rules relating to a specific activity or sport, such as a duration of regulation matches of the sport, training schedules for the sport, match schedules of the sport, or any combination of the foregoing. In some embodiments, outputs from machine learning model 160 can be considered unfiltered, and then run through a filter based on rules derived from real world considerations to obtain filtered results. For example, in some embodiments, timeline 170 can be a filtered timeline, and a process for generating timeline can comprise first generating an unfiltered timeline by use of machine learning model 160 as described above. Computing device 120 can then filter the unfiltered timeline by changing start times of individual events 171 within the unfiltered timeline to comply with filtering rules. The classification, which can also be considered filtering rules can comprise the real world considerations described above, such as possible durations for the events 171. For example, where the unfiltered timeline includes a "match" for a duration longer than a match could last according to the rules of the sport, computing device 120 can create filtered timeline 170 by delaying the beginning of the match within the timeline or moving earlier the beginning of an event 171 that immediately follows the match. In still further embodiments, machine learning model 160 can be trained in view of any of the foregoing real world considerations, classification rules, or rules relating to a specific sport and can weigh such considerations when grouping motion determinations for allocation into individual events 171.

FIG. 3B provides an alternative embodiment of visualizing the event classifications into different groups. The visualization of event classification may be based on a time-related parameter, such as a time duration of the event in relation to other classifications. FIG. 3B depicts "training," "exercise," and "match" as exemplary event classifications and a duration of time as the time-related parameter. For example, machine learning model 160 has classified the event determinations as 10 minutes of training, 15 minutes of exercise, and 20 minutes of match play.

In some embodiments, a second order analysis can be applied between the initial event classification or activity classification by machine learning model 160 and outputting final event classifications, such as by sending the final event classifications to visualizer 198. Such second order analysis can be within filtering component 196. Filtering component 196 according to various embodiments can be either an aspect of machine learning model 160 or a separate process or algorithm from machine learning model 160. Such second order analysis can comprise applying classification rules, such as the classification rules discussed above, to initial event classifications created by machine learning model 160 to refine the initial event classifications in view of other initial event classifications created from the same batch of motion determinations. Thus, the second order analysis can comprise changing initial event classifications within a plurality of event classifications into final event classifications by altering the initial event classifications in view of other initial event classifications within the same plurality of event classifications.

In some examples, the second order analysis can include reclassifying motion determinations, or portions of time, initially classified as "unknown" to match neighboring classifications of other events. In some such examples, individual motion determinations, small groups of motion determinations, or small portions of time initially classified as "unknown" and appearing within a larger window classified as a specific event (i.e., match, exercise, warmup, and so on) can be reclassified as the same event as the larger window. Pockets of "unknown" classification appearing in the middle of "match" classification can therefore be reclassified as "match," for example. "Unknown" initial event classifications appearing between two different types of other classifications can be reclassified according to what the classification rules show to be the likelier correct event classification. For example, where the classification rules include the fact that a particular sport has 90 minute matches preceded by a warmup phase, second order analysis according to some embodiments can convert initial event classifications including 25 minutes of warmup, 10 minutes of unknown following the warmup, and 85 minutes of match following the unknown into final, output event classifications including 30 minutes of warmup followed by 90 minutes of match.

In further examples, the second order analysis according to some embodiments can also convert non-"unknown" initial classifications into different final classifications according to classification rules. In further examples wherein the classification rules include the fact that a particular sport has 90 minute matches and training sessions that typically exceed an hour, the second order analysis can include converting initial event classifications including 15 minutes of exercise, 15 minutes of training following the exercise, and 90 minutes of match following the training to final, output event classifications including 30 minutes of warmup followed by 90 minutes of match. In still further examples within the same set of classification rules, the second order analysis can include converting initial event classifications including 10 minutes of exercise, 50 minutes of training following the exercise, and 30 minutes of match into a final, output event classification of a single 90 minute training session.

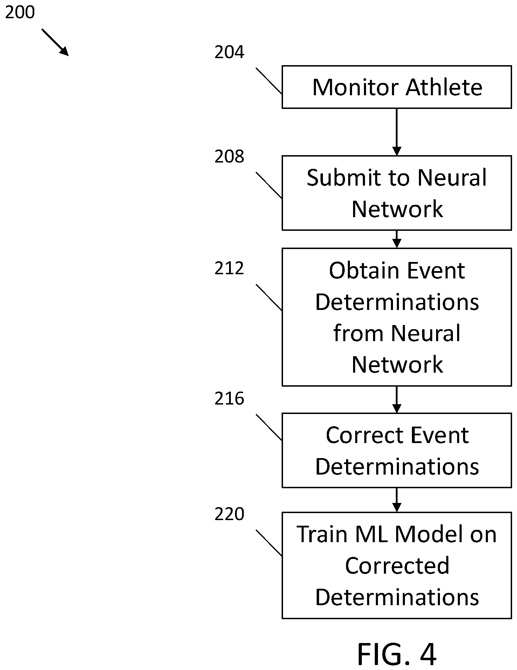

FIG. 4 shows a training method 200 according to some embodiments for training machine learning model 160 iteratively by identifying errors in outputs from machine learning model 160. A monitoring step 204 comprises monitoring athlete 118 with monitor 110 while athlete 118 participates in one or more of the predetermined events 171. Athlete 118 used for training method 200 can be considered a test athlete 118. Time during which monitoring step 204 is performed can be considered a test window. Motion classifications generated from motion data captured during monitoring step 204 can be considered test motion determinations.

A submitting step 208 comprises submitting the test motion determinations generated by monitor 110 from motion data captured during monitoring step 204 to machine learning model 160. An output step 212 comprises machine learning model 160 generating output, such as one or more event classifications, concerning what event 171 or events 171 among the plurality of predetermined events 171 the test athlete 118 participated in during monitoring step 204. The outputs generated in output step 204 can be considered test event classifications. In some embodiments, the output can comprise, for example, an output timeline 170 of events 171 participated in by athlete 118 during monitoring step 204. The output timeline 170 can be considered a test timeline 170.

A correcting step 216 comprises creating training data by correcting the test event classification obtained in output step 212. In some embodiments, the test event classifications can be corrected based on a record or records of what event 171 or events 171 the test athlete 118 participated in, and when athlete 118 began and ended participation in specific events 171, during the test window of monitoring step 204. In some embodiments, the record or records can comprise video footage of athlete 118 during monitoring step 204. In some embodiments, the training data can comprise a corrected timeline of events 171 the test athlete 118 participated in during monitoring step 204. In further embodiments, the training data comprises identifications of the corrections made to the test event classifications obtained during output step 212. A training step 220 comprises training machine learning model 160 based on the training data created during correcting step 216.

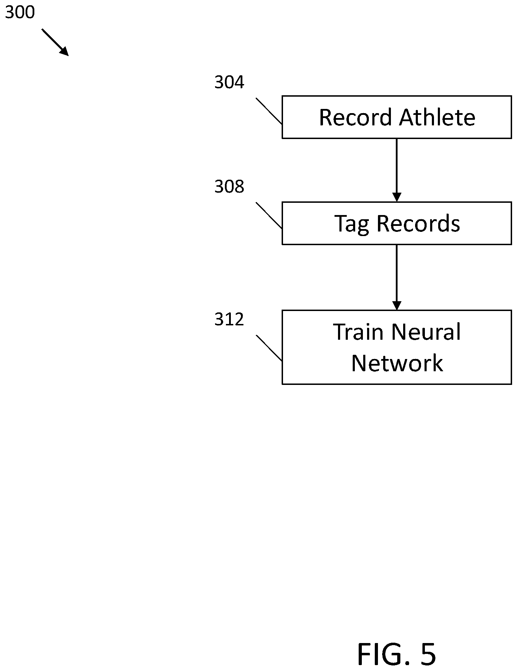

FIG. 5 shows a training method 300 according to some embodiments for training machine learning model 160 to identify events 171 athletes 118 participate in among a plurality of predetermined events 171. Within training method 300, a recording step 304 comprises recording activity of athletes 118 as athletes 118 participate in events 171 among the plurality of predetermined events 171. The events 171 occurring during recording step 304 can be considered sample events. In some embodiments, recording step 304 can comprise use of a monitor 110 by each participating athlete 118 to capture motion data from each participating motions of athlete 118. Motion classifications made by monitors 110 from motion data captured during the monitored participation by athlete 118 in the sample events can be considered sample motion determinations. In some embodiments, recording step 304 can further comprise acquiring video footage of athletes 118 participating in the sample events.

A tagging step 308 comprises tagging records generated in recording step 304 with indications of when sample events occurred. Tagging step 308 according to some embodiments can comprise tagging the video footage with start times of the sample events. Tagging step 308 according to some embodiments can comprise tagging the video footage with end times of the sample events. The tags can correspond to individual events among the plurality of predetermined events 171 described above. Accordingly, event type tags related to predetermined events such as, for example, movement training, ball training, match, warmup, and exercise can be applied. Tagging step 308 according to some embodiments can comprise tagging portions of the video footage with sample motion determinations generated from motion data acquired at the time of the events depicted in the portions of the video footage. Tagging step 308 according to some embodiments can comprise tagging portions of the video footage with activity tags corresponding to the activities of athletes 118 visible within the portions of the video footage.

Tagging step 308 according to some embodiments can comprise generating a training timeline from the sample motion determinations generated from motion data captured during recording step 304. Tagging step 308 can further comprise tagging the sample motion determinations within the training timeline with event type tags indicating the type of event 171 the relevant athlete 118 participated in during recording step 304. Thus, tagging records with indications of when sample events occurred can comprise tagging the sample motion determinations within the training timeline with event type tags. The applied tags can also comprise sub-event tags indicating shorter occurrences within the events. For example, motion training can comprise sub-events such as jogs and sprints. In further examples, ball training can comprise sub-events such as kicks of specific types. Thus, in some embodiments, movement training event tags can also include indications of sub-events such as, for example, 25 meter jog, 50 meter jog, 15 meter sprint, 25 meter sprint, or 50 meter sprint. In some embodiments, ball training event tags can also include indications of sub-events such as corner kick, free-kick, hard kick, penalty kick, and pass. In some embodiments, the inclusion of sub-event tags in training data for machine learning model 160 can help machine learning model 160 recognize sub-events and learn what sequences of sub-events tend to occur within individual events within the plurality of predetermined events 171.

Training step 312 comprises training machine learning model 160 on the tagged records created in tagging step 308 to recognize athletes' 118 participation in events 171 among the plurality of predetermined events 171. Training step 312 can therefore comprise training machine learning model 160 on the tagged footage created in tagging step 308 to recognize athletes' 118 participation in the events 171 among the plurality of predetermined events 171.

Training method 300 can be used to train an untrained machine learning model 160. That is, training method 300 does not require the machine learning model 160 being trained to already be prepared to generate output, such as in output step 212 of training method 200. However, when used on a machine learning model 160 prepared to generate outputs, training method 300 can be used in conjunction with training method 200 by implementing output step 212, correcting step 216, and training step 220 after training step 312.

Embodiments described herein also may be directed to computer program products comprising software stored on any computer useable medium. Such software, when executed in one or more data processing device, causes a data processing device(s) to operate as described herein. Embodiments described herein may employ any computer useable or readable medium. Examples of computer useable mediums include, but are not limited to, primary storage devices (for example, any type of random access memory), secondary storage devices (for example, hard drives, floppy disks, CD ROMS, ZIP disks, tapes, magnetic storage devices, and optical storage devices, MEMS, nano-technological storage device, etc.).

It is to be appreciated that the Detailed Description section, and not the Summary and Abstract sections, is intended to be used to interpret the claims. The Summary and Abstract sections may set forth one or more but not all exemplary embodiments of the present disclosure as contemplated by the inventor(s), and thus, are not intended to limit the present invention(s) and the appended claims in any way.

The present invention(s) have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention(s) that others can, by applying knowledge within the skill of the art, readily modify and/or adapt for various applications such specific embodiments, without undue experimentation, without departing from the general concept of the present invention(s). Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the concepts of the present disclosure should not be limited by any of the above-described exemplary embodiments, but should be defined only in accordance with the following claims and their equivalents.

In the following, further examples are described to facilitate the understanding of the invention.
1. A method of determining an event participated in by an athlete, the method comprising:
   receiving, at a computing device, a plurality of motion determinations generated based on motion data captured from motion data of the athlete during a monitoring window, wherein the motion determinations comprise at least one of an action performed by the athlete and performance metrics of the athlete;
   classifying, by use of a machine learning model stored on the computing device and based on the motion determinations, an event based at least in part on the plurality of motion determinations, wherein the event represents a classification of the plurality of motion determinations;
   generating a graphical user interface visualizing the event in relation to a time-related parameter.
2. The method of example 1, comprising: generating, by the computing device, a timeline of events participated in by the athlete based at least in part on a set of motion determinations comprising the plurality of motion determinations by applying the machine learning model to the set of motion determinations.
3. The method of example 2, wherein generating the timeline of events comprises classifying individual motion determinations among the plurality of motion determinations as being generated from motion data captured during individual events among the timeline of events.
4. The method of example 2, wherein:
   the timeline of events participated in by the athlete is an output timeline,
   the method comprises, before the receiving step, training the machine learning model to identify events athletes participate in by submitting training timelines to the machine learning model, and
   each training timeline comprises a plurality of sample motion determinations and indications of when sample events occurred.
5. The method of example 4, wherein the indications of when sample events occurred include event type tags associated with sample motion determinations among the plurality of sample motion determinations.
6. The method of example 2, wherein the timeline is a filtered timeline, and generating the timeline comprises:
   generating an unfiltered timeline of events participated in by the athlete based in part on the set of motion determinations by applying the machine learning model to the set of motion determinations; and
   filtering the unfiltered timeline of events by changing start times of individual events within the timeline of events to comply with filtering rules.
7. The method of example 6, wherein the filtering rules comprise possible durations for events among a plurality of predetermined events.
8. The method of example 1, wherein the plurality of predetermined events comprises exercise, training for a sport, and a match of the sport.
9. The method of example 1, comprising training the machine learning model to classify events participated in based on motion determinations, wherein the training comprises:
   creating a plurality of test motion determinations based on motions of a test athlete during a test window;
   using the machine learning model to output a test event classification of which event among the plurality of predetermined events the test athlete participated in during the test window based on the plurality of test motion determinations; and
   correcting the test event classification based on a record of what event the test athlete participated in during the test window.
10. The method of example 1, comprising determining, by the computing device, a role of the athlete in a team sport based at least in part on the plurality of motion determinations by applying the machine learning model to the plurality of motion determinations.
11. The method of example 1, wherein the motion determinations comprise actions performed by the athlete, and the actions performed by the athlete comprise any one or any combination of a kick, a step, dribbling a ball, and running.
12. The method of example 1, wherein the motion determinations comprise performance metrics, and the performance metrics comprise any one or any combination of distance traveled, travel speed, and kick force.
13. The method of example 1, wherein the classifying, by use of the machine learning model, which event among a plurality of predetermined events the athlete participated in during the monitoring window is further based on video footage of the athlete during the monitoring window.
14. The method of example 13, further comprising training the machine learning model to classify events participated in by athletes based on video footage, wherein the training comprises:
   creating tagged footage by tagging training video footage of athletes participating in events among the plurality of predetermined events with start times of the events among the plurality of predetermined events; and
   training the machine learning model on the tagged footage to recognize participation in the events among the plurality of predetermined events.
15. A system comprising:
   a wearable sensor configured to measure motion of a wearer of the wearable sensor;
   a controller configured to generate motion determinations from motion data captured by the wearable sensor, wherein the motion determinations comprise either or both of actions performed by the wearer and performance metrics of the wearer; and
   a computing device comprising a processor and a non-transitory computer readable medium, wherein the non-transitory computer readable medium carries instructions that, when read by the processor, cause the processor to classify which event among a plurality of predetermined events the wearer participated in while the motion data were captured by the wearable sensor based at least in part on a plurality of the motion determinations.
16. The system of example 15, wherein the wearable sensor is configured to be integrated into an article of wear.
17. The system of example 15, wherein the article of wear comprises a shoe.
18. The system of example 15, comprising a wearable monitor that comprises the wearable sensor and the controller.
19. The system of example 15, wherein the computing device is remote from the wearable sensor.
20. The system of example 19, wherein the computing device comprises any one or any combination of a smart device, a laptop computer, a desktop computer, or a cloud computing system.

## Claims

1. A method of determining an event participated in by an athlete, the method comprising:
receiving, at a computing device, a plurality of motion determinations generated based on motion data captured from motion data of the athlete during a monitoring window, wherein the motion determinations comprise at least one of an action performed by the athlete and performance metrics of the athlete;
classifying, by use of a machine learning model stored on the computing device and based on the motion determinations, an event based at least in part on the plurality of motion determinations, wherein the event represents a classification of the plurality of motion determinations;
generating a graphical user interface visualizing the event in relation to a time-related parameter.

2. The method of claim 1, comprising: generating, by the computing device, a timeline of events participated in by the athlete based at least in part on a set of motion determinations comprising the plurality of motion determinations by applying the machine learning model to the set of motion determinations.

3. The method of claim 2, wherein generating the timeline of events comprises classifying individual motion determinations among the plurality of motion determinations as being generated from motion data captured during individual events among the timeline of events.

4. The method of claim 2 or 3, wherein:
the timeline of events participated in by the athlete is an output timeline,
the method comprises, before the receiving step, training the machine learning model to identify events athletes participate in by submitting training timelines to the machine learning model, and
each training timeline comprises a plurality of sample motion determinations and indications of when sample events occurred, wherein the indications of when sample events occurred include preferably event type tags associated with sample motion determinations among the plurality of sample motion determinations.

5. The method of one of claims 2 to 4, wherein the timeline is a filtered timeline, and generating the timeline comprises:
generating an unfiltered timeline of events participated in by the athlete based in part on the set of motion determinations by applying the machine learning model to the set of motion determinations; and
filtering the unfiltered timeline of events by changing start times of individual events within the timeline of events to comply with filtering rules, wherein the filtering rules comprise preferably possible durations for events among a plurality of predetermined events.

6. The method of one of the preceding claims, wherein the plurality of predetermined events comprises exercise, training for a sport, and a match of the sport.

7. The method of one of the preceding claims, comprising training the machine learning model to classify events participated in based on motion determinations, wherein the training comprises:
creating a plurality of test motion determinations based on motions of a test athlete during a test window;
using the machine learning model to output a test event classification of which event among the plurality of predetermined events the test athlete participated in during the test window based on the plurality of test motion determinations; and
correcting the test event classification based on a record of what event the test athlete participated in during the test window.

8. The method of one of the preceding claims, comprising determining, by the computing device, a role of the athlete in a team sport based at least in part on the plurality of motion determinations by applying the machine learning model to the plurality of motion determinations.

9. The method of one of the preceding claims, wherein the motion determinations comprise actions performed by the athlete, and the actions performed by the athlete comprise any one or any combination of a kick, a step, dribbling a ball, and running.

10. The method of one of the preceding claims, wherein the motion determinations comprise performance metrics, and the performance metrics comprise any one or any combination of distance traveled, travel speed, and kick force.

11. The method of one of the preceding claims, wherein the classifying, by use of the machine learning model, which event among a plurality of predetermined events the athlete participated in during the monitoring window is further based on video footage of the athlete during the monitoring window, further comprising preferably training the machine learning model to classify events participated in by athletes based on video footage, wherein the training comprises:
creating tagged footage by tagging training video footage of athletes participating in events among the plurality of predetermined events with start times of the events among the plurality of predetermined events; and
training the machine learning model on the tagged footage to recognize participation in the events among the plurality of predetermined events.

12. A system comprising:
a wearable sensor configured to measure motion of a wearer of the wearable sensor;
a controller configured to generate motion determinations from motion data captured by the wearable sensor, wherein the motion determinations comprise either or both of actions performed by the wearer and performance metrics of the wearer; and
a computing device comprising a processor and a non-transitory computer readable medium, wherein the non-transitory computer readable medium carries instructions that, when read by the processor, cause the processor to classify which event among a plurality of predetermined events the wearer participated in while the motion data were captured by the wearable sensor based at least in part on a plurality of the motion determinations.

13. The system of claim 12, wherein the wearable sensor is configured to be integrated into an article of wear.

14. The system of claim 12 or 13, wherein the article of wear comprises a shoe.

15. The system of one of claims 12 to 14, comprising a wearable monitor that comprises the wearable sensor and the controller, and/or wherein the computing device is remote from the wearable sensor, wherein the computing device comprises preferably any one or any combination of a smart device, a laptop computer, a desktop computer, or a cloud computing system.
